# EUROPEAN PATENT APPLICATION

(11) **EP 4 487 758 A1**
(43) Date of publication of application: **08.01.2025**
(21) Application number: 22958471.9
(22) Date of filing: 16.09.2022
(51) Int. Cl.: A61B 5/00

(54) **SIGNAL MEASUREMENT METHOD AND CIRCUIT**

(71) Applicant: Shenzhen Shokz Co., Ltd., Shenzhen, Guangdong 518108 (CN)
(72) Inventor: ZHOU, Xin, Shenzhen, Guangdong 518108 (CN); ZHANG, Yuxiang, Shenzhen, Guangdong 518108 (CN); SU, Lei, Shenzhen, Guangdong 518108 (CN); LI, Meiqi, Shenzhen, Guangdong 518108 (CN); LIU, Jia, Shenzhen, Guangdong 518108 (CN); LIAO, Fengyun, Shenzhen, Guangdong 518108 (CN); QI, Xin, Shenzhen, Guangdong 518108 (CN)
(74) Representative: Wang, Bo
(86) International application number: PCT/CN2022/119261
(87) International publication number: WO 2024/055280

(57) **Abstract**

Embodiments of the present disclosure disclose a signal measurement method and circuit, comprising: receiving a detection signal, the detection signal reflecting a difference between two contact impedances, each of which is a contact impedance between one of two electrodes (111, 112) and a human body, and the two electrodes (111, 112) being affixed to the human body and being configured to collect a physiological signal of the human body; and determining an indicator parameter value reflecting a quality of the physiological signal based on the detection signal. The signal measurement method and circuit provided in the present disclosure can detect the contact impedance between each of the two electrodes (111, 112) and the human body in real-time, and determine, based on the contact impedance, state between each of the two electrodes (111, 112) and the human body, and thus ensure that a physiological signal collected by the electrodes have a high quality.

## Description

### TECHNICAL FIELD

The present disclosure relates to the field of signal detection technology, and in particular, to a signal measurement method and a signal measurement circuit.

### BACKGROUND

With the growing interest in physiological health, there is an increasing demand for devices for monitoring physiological signals. For example, information from electromyogram (EMG) signals can be utilized to make a clinical diagnosis of a wide range of disorders such as muscle fatigability, myasthenia gravis, myasthenia gravis, and myasthenia gravis. The quality of the physiological signals collected during the monitoring process is one of the most important factors affecting the accuracy of the monitoring.

When collecting physiological signals, if the electrodes don't fit the human skin well, or if the skin is nearly insulated in dry environments, it will lead to poor quality of the collected physiological signals, making it difficult to monitor accurately.

Therefore, it is desired that a method can be proposed to detect fitting states between the electrodes and the human skin in real time and to ensure the quality of the collected physiological signals.

### SUMMARY

Embodiments of the present specification provide a signal measurement method, comprising: receiving a detection signal, the detection signal reflecting a difference between two contact impedances, each of which is a contact impedance between one of two electrodes and a human body, and the two electrodes being affixed to the human body and being configured to collect a physiological signal of the human body; and determining an indicator parameter value reflecting a quality of the physiological signal based on the detection signal.

In some embodiments, the indicator parameter value includes a magnitude value of the detection signal at a preset moment and/or a fluctuation of magnitude values of the detection signal within a preset period.

In some embodiments, the detection signal is periodic, and the indicator parameter value is a representation of the detection signal corresponding to at least one cycle.

In some embodiments, the method further comprises: in response to determining that the indicator parameter value satisfies a preset indicator condition, displaying the physiological signal, wherein the preset indicator condition is that a fluctuation within a preset duration is less than a preset threshold value.

In some embodiments, the method further comprises: in response to determining that the indicator parameter value does not satisfy the preset indicator condition, not displaying the physiological signal.

In some embodiments, the method further comprises: in response to determining that the indicator parameter value does not satisfy the preset indicator condition, determining feedback information including the indicator parameter value; and determining a target physiological signal by invoking a signal processing algorithm based on the feedback information and processing the physiological signal.

In some embodiments, the signal processing algorithm includes at least one of: a signal filtering algorithm, a wavelet transform algorithm, or a machine learning algorithm.

In some embodiments, the invoking the signal processing algorithm based on the feedback information includes determining a weight of the physiological signal based on the indicator parameter value of the feedback information; and invoking, based on a threshold range in which the weight is located, the signal processing algorithm corresponding to the threshold range.

In some embodiments, the invoking the signal processing algorithm based on the feedback information includes: invoking the signal processing algorithm corresponding to the feedback information by using the feedback information as an input of a trained machine learning model and running the trained machine learning model.

In some embodiments, the method further comprises: in response to determining that the indicator parameter value does not satisfy the preset indicator condition, obtaining a historical physiological signal of the human body; and displaying an alternative physiological signal based on the historical physiological signal and the physiological signal.

In some embodiments, the method further comprises: generating quality information for assessing the quality of the physiological signal based on a fluctuation range of the indicator parameter value within the preset duration.

In some embodiments, the method further comprises: generating parameter information for evaluating fitting states of the two electrodes based on the indicator parameter value.

In some embodiments, the method further comprises: in response to determining that the indicator parameter value is not within a target magnitude value range, outputting an anomaly alert message.

In some embodiments, the target magnitude value range includes a first magnitude value range, the signal measurement method further comprising: in response to determining that the indicator parameter value is within the first magnitude value range, outputting a first indication message, the first indication message being configured to indicate a user to move.

In some embodiments, the target magnitude value range includes a second magnitude value range, the signal measurement method further comprising: in response to determining that the indicator parameter value is within the second magnitude value range, outputting a second indication message, the second indication message being configured to instruct a user to adjust fitting states of the two electrodes.

Example embodiments of the present disclosure provide a signal measurement circuit, comprising: two electrodes, configured to affix to a human body to collect a physiological signal of the human body; an alternating current (AC) excitation source electrically connected to each of the two electrodes, the AC excitation source being configured to provide an excitation signal to generate a detection signal reflecting a contact impedance between each of the electrodes and the human body; and a processing circuit, configured to determine a difference between detection signals corresponding to the two electrodes for evaluating the physiological signal.

In some embodiments, the processing circuit includes a gain circuit, and the detection signals corresponding to the two electrodes are designated as an input of the gain circuit for differential amplification.

In some embodiments, the AC excitation source is coupled to a corresponding electrode through a resistance voltage divider, and the resistance voltage divider divides a voltage of the AC excitation source to generate a corresponding detection signal.

In some embodiments, a frequency of the AC excitation source is greater than 250 Hz.

In some embodiments, a ratio of an impedance of the resistance voltage divider at the frequency of the AC excitation source to an impedance of the resistance voltage divider at a frequency less than 400 Hz is not greater than 1.6.

In some embodiments, impedances of two resistance voltage dividers at the frequency of the AC excitation source are equal.

In some embodiments, the processing circuit further includes an analog-to-digital converter disposed at an output end of the gain circuit, and the analog-to-digital converter is configured to perform an analog-to-digital conversion on the physiological signal and the detection signals.

In some embodiments, the processing circuit further includes a low-pass filter, and the low-pass filter is configured to extract the physiological signal that is analog-to-digital converted by the analog-to-digital converter; and the processing circuit further includes a band-pass filter or a high-pass filter, and the band-pass filter or the high-pass filter is configured to extract a difference between the detection signals that are analog-to-digital converted by the analog-to-digital converter.

In some embodiments, the AC excitation source is further configured to provide a second excitation signal at a different frequency from that of the excitation signal to generate a second detection signal, and the second detection signal is configured to reflect a contact impedance between each of the two electrodes and the human body at another frequency.

In some embodiments, the processing circuit evaluates body composition information of the human body based on the detection signals and the second detection signal.

The methods and circuits provided in embodiments of the present disclosure can detect the contact impedance between one of the two electrodes and the human body in real time, and determine the fitting state between one of the two electrodes and the human body based on the contact impedance, thereby ensuring the quality of the physiological signals collected by the electrodes.

Additional features will be set forth in part in the following description and will become apparent to those skilled in the art by consulting the following and the accompanying drawings or may be appreciated by the generation or operation of examples. Features of the present disclosure may be realized and obtained by practicing or using aspects of the methods, tools, and combinations set forth in the following detailed examples.

### BRIEF DESCRIPTION OF THE DRAWINGS

The present disclosure will be further illustrated by way of exemplary embodiments, which will be described in detail by means of the accompanying drawings. These embodiments are not limiting, and in these embodiments, the same numbering denotes the same structure, where:
FIG. 1 is a schematic diagram illustrating a signal measurement circuit according to some embodiments of the present disclosure;
FIG. 2 is a schematic diagram illustrating a signal measurement circuit according to other embodiments of the present disclosure;
FIG. 3 is a schematic diagram illustrating a signal measurement circuit according to some other embodiments of the present disclosure;
FIG. 4 is a schematic diagram illustrating a processing circuit according to some embodiments of the present disclosure;
FIG. 5 is a flowchart illustrating a signal measurement method according to some embodiments of the present disclosure;
FIG. 6A and FIG. 6B are waveform diagrams of signal measurement results according to some embodiments of the present disclosure;
FIG. 7A is a flowchart illustrating a signal measurement method according to some other embodiments of the present disclosure;
FIG. 7B is a flowchart illustrating a signal measurement method according to some other embodiments of the present disclosure; and
FIG. 8 is a flowchart illustrating a signal measurement method according to some other embodiments of the present disclosure.

### DETAILED DESCRIPTION

In order to more clearly illustrate the technical solutions of the embodiments of the present disclosure, the following will briefly describe the accompanying drawings to be used in the description of the embodiments. Obviously, the accompanying drawings in the following description are only some examples or embodiments of the present disclosure, and it is possible for a person of ordinary skill in the art to apply the present disclosure to other similar scenarios in accordance with these drawings without creative labor. It should be understood that these exemplary embodiments are given only to enable those skilled in the art to better understand and thus realize the present disclosure, and are not intended to limit the scope of the present disclosure in any way. Unless obviously obtained from the context or the context illustrates otherwise, the same numeral in the drawings refers to the same structure or operation.

As shown in the present disclosure and in the claims, unless the context clearly suggests an exception, the words "a," "an," "one," "one kind," and/or "the" do not refer specifically to the singular, but may also include the plural. In general, the terms "include," "comprise," and/or "including," "comprising," "includes," and/or "comprises," merely prompt to include steps and elements that have been clearly identified, and these steps and elements do not constitute an exclusive listing and may also include other steps or elements.

In the description of the present specification, it is to be understood that the terms "first," "second", or the like are used only for descriptive purposes and are not to be understood as indicating or implying relative importance or implicitly specifying the number of technical features indicated. Thereby, the limitation that a feature such as "first," "second", or the like may expressly or implicitly include at least one such feature. In the description of the present disclosure, "plurality" means at least two, e.g., two, three, or the like, unless explicitly and specifically limited otherwise.

A signal measurement method and circuit described in embodiments of the present disclosure is applied to wearable products, e.g., the wearable products include apparel, backpacks, helmets, eyeglasses, headsets, VR/AR devices, or the like. In some embodiments, the signal measurement circuit includes two electrodes configured to affix to a human body to collect a physiological signal of the human body. In some embodiments, in order for the physiological signal collected by the electrodes to accurately reflect a physiological state of the human body, the signal measurement circuit simultaneously monitors a fitting state between each of the two electrodes and the human body to evaluate a quality of the physiological signal. In some embodiments, the physiological signal is subsequently processed in different ways for different fitting states between each of the two electrodes and the human body so as to well reflect the physiological state of the human body. For example, when the signal measurement circuit detects that the fitting state between each of the two electrodes and the human body is good (well fitted), a corresponding physiological signal can be directly configured to evaluate the physiological state of the human body, and when the signal measurement circuit detects that the fitting state between each of the two electrodes and the human body is bad (poor contact), the corresponding physiological signal is discarded or calibrated by a certain algorithm before being configured to evaluate the physiological state of the human body.

In some embodiments, by means of the signal measurement method and circuit described in the embodiments of the present disclosure, it is further possible to perform wear state analysis, warm-up state analysis, size fit analysis, garment life analysis, movement monitoring, skin condition analysis, body composition analysis, or the like based on an evaluation result of the physiological signal. The signal measurement method and circuit provided by the embodiments of the present disclosure are described in detail below in conjunction with the accompanying drawings.

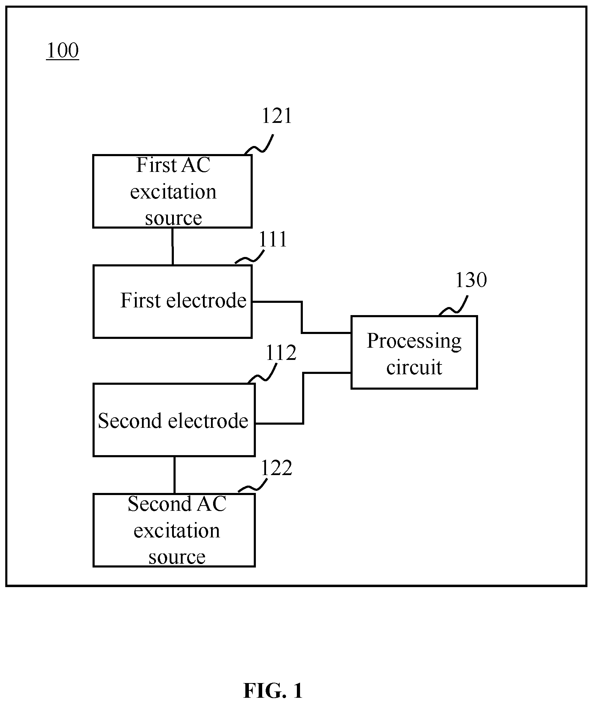

FIG. 1 is a schematic diagram illustrating a signal measurement circuit according to some embodiments of the present disclosure.

Referring to FIG. 1, in some embodiments, a signal measurement circuit 100 includes two electrodes (a first electrode 111 and a second electrode 112 as shown in FIG. 1), two alternating current (AC) excitation sources (a first AC excitation source 121 and a second AC excitation source 122 as shown in FIG. 1) electrically connected to the two electrodes, respectively, and a processing circuit 130.

Electrodes (e.g., the first electrode 111 and the second electrode 112) refer to circuit elements for contacting other objects to input or derive voltage (current). In some embodiments, the electrodes are configured to affix to a human body to collect a physiological signal (e.g., an EMG signal, etc.) of the human body. In some embodiments, the physiological signal is a signal that is detected to be able to reflect a state of the body. For example, the physiological signal includes a respiratory signal, an electrocardiogram (ECG) signal, an electromyogram (EMG) signal, a blood pressure signal, a temperature signal, or the like. In some embodiments, a frequency range of the physiological signal is in a range of 0.05 Hz to 2 kHz, and a frequency range of the ECG signal is in a range of 0.05 Hz to 100 Hz, and a frequency range of the EMG signal is in a range of 10 Hz to 850 Hz. In order to detect a muscle state of the human body, in some embodiments, two electrodes are affixed to a same muscle, so that a difference between EMG signals collected by the two electrodes may reflect a state of the muscle.

In some embodiments, a contact surface between the electrode with the body is planar or non-planar. In some embodiments, the electrode is in contact with the body in a form of a flexible patch. The flexible patch may be a regular shape such as a circle, an oval, a rectangle, a diamond, or other irregular shape. In some embodiments, the electrode is made of a metallic conductive material (e.g., copper, aluminum, nickel, chromium, alloys, etc.) and a non-metallic conductive material (e.g., conductive plastics, conductive rubbers, conductive silicones, conductive fabrics, etc.).

In some embodiments, the signal measurement circuit 100 also includes one or more reference ground electrodes (not shown in FIG. 1) that may serve as a reference ground in the signal measurement circuit 100. In some embodiments, the one or more reference ground electrodes are configured to affix to the human body, and each of the reference ground electrodes cooperates with one or more electrodes, which in turn allows an excitation signal generated by the AC excitation source to flow through the human body to the one or more reference ground electrodes to form a closed-loop circuit. For example, an excitation signal generated by the first AC excitation source 121 flows to the first electrode 111, a contact impedance between the first electrode 111 and the human body, tissues of the human body, and the one or more reference ground electrodes in turn, thereby forming a closed-loop circuit. In some embodiments, a plurality of reference ground electrodes also cooperate with one or more electrodes, such that an excitation signal generated by the AC excitation source may flow to the plurality of reference ground electrodes via a plurality of parts of the human body to form a closed-loop circuit. For example, the excitation signal generated by the first AC excitation source 121 flows sequentially to the first electrode 111, the contact impedance between the first electrode 111 and the human body, tissues of the human body corresponding to the plurality of reference ground electrodes, and the plurality of reference ground electrodes, thereby forming a closed-loop circuit. In some embodiments, the one or more reference ground electrodes are provided in a flat and stable (small in movement amplitude) part of the body, such as the back, waist, legs, etc. of the human body, so as to effectively reduce noise.

The AC excitation source is an energy supply circuit element that provides an AC excitation signal. In some embodiments, the AC excitation source is configured to provide the AC excitation signal to the electrode to generate a detection signal reflecting a contact impedance between the electrode and the body. For example, the first AC excitation source 121 is electrically connected to the first electrode 111, and the second AC excitation source 122 is electrically connected to the second electrode 112. The first AC excitation source 121 and the second AC excitation source 122 are configured to provide an AC excitation signal for the first electrode 111 and the second electrode 112, respectively, to generate detection signals related to a contact impedance corresponding to the first electrode 111 and the second electrode 112, respectively.

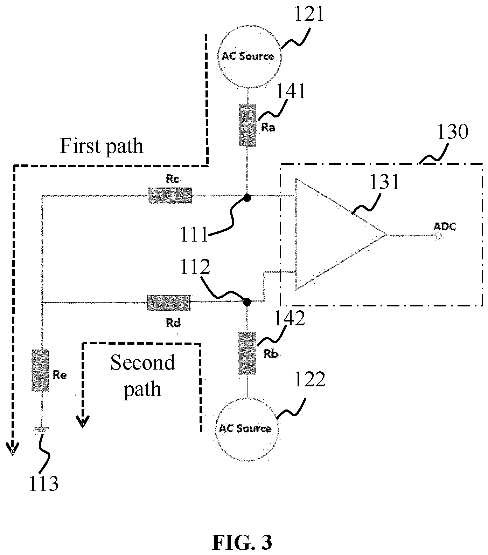

In some embodiments, the contact impedance is an impedance generated by the human body coming into contact with an electrode of a signal measurement device, and an impedance value of the contact impedance is affected by a dry state of the skin of the human body in contact with a resistor, and a contact state of the human body in contact with the skin. The impedance value of the contact impedance may affect a quality of a collected physiological signal. For example, the drier the skin is, the greater the impedance value of the contact impedance is, resulting in a poorer quality of the collected physiological signal; conversely, the wetter the skin is, the less the impedance value of the contact impedance is, and the better the quality of the collected physiological signal. Furthermore, for example, poor contact between the human body and the skin results in a greater impedance value of the contact impedance, making the quality of the physiological signal worse. In some embodiments, the excitation signals are provided for the two electrodes, respectively, and a detection signal corresponding to the contact impedance of each of the two electrodes is obtained, respectively. The two detection signals are subsequently processed, by way of differencing, summing, or other manners. Specific descriptions of the contact impedance and the detection signal can be found in other parts of the present disclosure, e.g., FIG. 3 and its description.

When the detection signal and the EMG signal are collected at the same time, in order to be able to differentiate between the detection signal and the EMG signal in subsequent processing, in some embodiments, the AC excitation source is configured to provide the excitation signal, so that there is a difference between a frequency of the detection signal and a frequency of the EMG signal, and based on the difference between the frequency of the detection signal and the frequency of the EMG signal, the detection signal and the EMG signal are subsequently extracted, respectively, thereby avoiding interference between the detection signal and the EMG signal. In some embodiments, the AC excitation source is a current source or a voltage source. In some embodiments, a current intensity of the AC excitation source is less than 1 mA. Furthermore, in some embodiments, the current intensity of the AC excitation source is less than 100 µA. Furthermore, in some embodiments, the current intensity of the AC excitation source is less than 10 µA. In some embodiments, a voltage intensity of the AC excitation source is in an intensity range of 10 µV to 3.3 V. Furthermore, in some embodiments, the voltage intensity of the AC excitation source is in an intensity range of 100 mV to 9 V. In some embodiments, the AC excitation source generates excitation signals of at least two frequencies, respectively, and provides excitation signals of a same or different frequencies to the first electrode 111 and the second electrode 112.

It should be noted that in some embodiments of the present disclosure, the signal measurement circuit 100 includes two or more electrodes, and the first electrode 111 and the second electrode 112 refer to any two of those two or more electrodes. In some embodiments, a portion of the two or more electrodes is connected with the first AC excitation source 121, and a remaining portion is connected with the second AC excitation source 122. In some embodiments, each of the two or more electrodes is connected to an AC excitation source, respectively. In this case, the first AC excitation source 121 and the second AC excitation source 122 refer to any two different AC excitation sources of a plurality of AC excitation sources. In some embodiments, the first AC excitation source 121 and the second AC excitation source 122 are replaced by a single AC excitation source. For example, the single AC excitation source is connected to a plurality of electrodes via a plurality of branches to provide excitation signals to a plurality of electrodes. One electrode is provided for each of the plurality of branches.

The processing circuit 130 refers to a circuit having certain signal processing functions, such as amplification, rectification, A/D conversion, filtering, logic processing, or the like. In some embodiments, the processing circuit 130 may include a processor, such as a Digital Signal Processor (DSP) chip, a Central Processing Unit / Processor (CPU), a Microcontroller Unit (MCU), or the like. In some embodiments, the processing circuit 130 is configured to process the detection signal and evaluate the physiological signal or to determine an indicator parameter value reflecting the quality of the physiological signal. For example purposes only, in some embodiments, the processing circuit 130 is configured to process a detection signal corresponding to a particular electrode alone, and in doing so, evaluate a physiological signal collected by the electrode or determine an indicator parameter value reflecting the quality of the physiological signal. Exemplarily, the processing circuit 130 may amplify the detection signal corresponding to the first electrode 111 and then perform an analog-to-digital (A/D) conversion on the detection signal. The detection signal in this case may individually reflect a contact impedance between the first electrode 111 and the human body, and thus reflect a fitting state of the first electrode 111 with the human body. Furthermore, for example, the processing circuit 130 may filter the detection signal corresponding to the first electrode 111 to differentiate an EMG signal collected by the first electrode 111. In some embodiments, the processing circuit 130 may combine detection signals corresponding to a plurality of electrodes to comprehensively evaluate physiological signals collected by the plurality of electrodes or indicator parameter values reflecting the quality of the physiological signals.

Exemplarily, the processing circuit 130 may perform operational amplification (e.g., differential amplification) on the detection signals corresponding to the first electrode 111 and the second electrode 112, and then perform the analog-to-digital (A/D) conversion on amplified results. The detection signal after differential amplification may synthesize a difference in contact impedance between the two electrodes and the human body, thus reflecting whether both electrodes fits well (or affix well) with the human body. Furthermore, the processing circuit 130 may filter the amplified results to differentiate EMG signals collected by the first electrode 111 and the second electrode 112. In some embodiments, the processing circuit 130 may directly determine a difference between the detection signals corresponding to the first electrode 111 and the second electrode 112, and then evaluate the physiological signals based on the difference.

It is noted that processing functions of the processing circuit 130 may include, but are not limited to, the above enumerations. For example, in some embodiments, the processing circuit 130 may also determine a sum of the detection signals corresponding to the first electrode 111 and the second electrode 112, and then evaluate the physiological signals based on the sum. In some cases, evaluating the physiological signal based on the difference between the detection signals (or a value after differential amplification by an operational amplifier) better reflects the signal quality compared to the evaluation based on a summation of the detection signals. Therefore, in the embodiments of the present disclosure, the signal measurement circuit 100 is primarily described using the difference between the detection signals (or the value after differential amplification by an operational amplifier) as an example.

In some embodiments, the evaluation of the physiological signal by the processing circuit 130 may include, among other things, determining the quality of the physiological signal and evaluating fitting states between the first electrode 111 and/or the second electrode 112 and the human body.

In some embodiments, the signal measurement circuit 100 is applied to a smart wearable device. In some embodiments, the smart wearable device may include a smart bracelet, smart footwear, smart glasses, a smart helmet, a smart watch, a smart garment, a smart backpack, a smart accessory, or the like, or any combination thereof. Taking an intelligent garment (e.g., a fitness garment or sportswear) as an example, the garment may include two or more electrodes, and the two or more electrodes are arranged in different parts of the garment for affixing with different parts of the human body to collect physiological signals from different parts. For example, the two or more electrodes are disposed on the chest, back, elbow, leg, abdomen, wrist, or the like. The processing circuit 130 may collect physiological signals collected by any two or two electrodes with a particular association (e.g., two electrodes affixed to the same muscle to receive EMG signals from a same muscle) and evaluate the quality of the physiological signals based on a difference between the physiological signals collected by the any two electrodes or the two electrodes with a particular association, or value a fitting state between the any two or the two electrodes with the human body.

In some embodiments, the smart wearable device may further include an output device, such as a speaker, a display, etc., by which a result of the evaluation of the physiological signal collected by the electrodes by the processing circuit 130 is output. In some embodiments, the wearable device may also be connected to other terminals (e.g., cell phones, computers, etc.) by wired or wireless (e.g., Bluetooth, Wi-Fi, etc.) means, and the processing circuit 130 may send the physiological signal and/or the result of the evaluation of physiological signal collected by the electrodes to the other terminals and output the result via other terminals (e.g., by displaying the result via a display screen or broadcasting the result via a speaker).

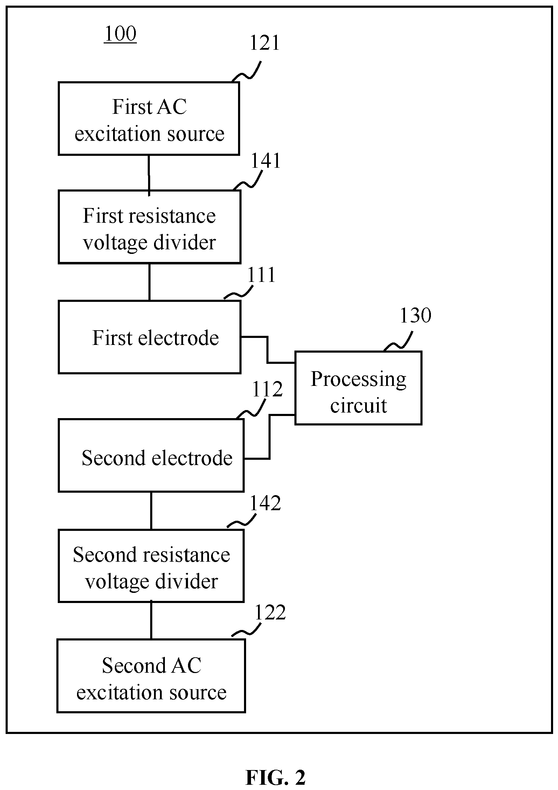

FIG. 2 is a schematic diagram illustrating a signal measurement circuit according to some other embodiments of the present disclosure.

In some embodiments, each AC excitation source is coupled to a corresponding electrode through a resistance voltage divider, and the resistance voltage divider divides the AC excitation source to form a corresponding detection signal. In some embodiments, the detection signal is viewed as a voltage across two ends of a contact impedance, and the detection signal may reflect a magnitude of a contact impedance between an electrode and the human skin. In some embodiments, when resistance voltage-divided impedances corresponding to two electrodes have the same parameter, a difference in detection signals corresponding to the two electrodes may reflect a difference in contact impedances between each of the two electrodes and the human skin. For example, the greater the difference between the two detection signals indicates the greater the difference between the two contact impedances corresponding to the detection signals, and the worse the quality of a corresponding EMG signal. Conversely, the lesser the difference between the two detection signals indicates that the less the difference between the two contact impedances corresponding to the detection signals, and the better the quality of the corresponding EMG signal. In some embodiments, detection signals of different frequencies may also be configured to reflect body composition information of a human body, such as a body fat rate, a bone density, a body fluid content, or the like. For a specific description of the detection signal, reference may be made to other parts of the present disclosure, for example, FIG. 3 and its description.

According to FIG. 2, in some embodiments, the signal measurement circuit 100 may include a first resistance voltage divider 141 coupled between the first AC excitation source 121 and the first electrode 111, and a second resistance voltage divider 142 coupled between the second AC excitation source 122 and the second electrode 112. After emitting an excitation signal from the first AC excitation source 121, the excitation signal may flow through the first resistance voltage divider 141, and after a voltage of the excitation signal is divided by the first resistance voltage divider 141, the excitation signal may flow to the human body through the first electrode 111, and finally flow to a reference ground electrode, thereby forming a first path. The first resistance voltage divider 141 may divide the voltage of the excitation signal provided by the first AC excitation source 121, thereby forming a detection signal corresponding to the first electrode 111. Similarly, after emitting an excitation signal from the second AC excitation source 122, the excitation signal may flow through the second resistance voltage divider 142, and after a voltage of the excitation signal is divided by the second resistance voltage divider 142, the excitation signal may flow to the human body through the second electrode 112, and finally to the reference ground electrode, thereby forming a second path. The second resistance voltage divider 142 may divide the voltage of the excitation signal provided by the second AC excitation source 122 to form a detection signal corresponding to the second electrode 112.

In some embodiments, the first path and the second path may have a certain symmetry. Specifically, i.e., the first AC excitation source 121 and the second AC excitation source 122 may have same parameters, and the first resistance voltage divider 141 and the second resistance voltage divider 142 may have same parameters. For example, the first AC excitation source 121 and the second AC excitation source 122 may generate excitation signals having the same frequency, and the first resistance voltage divider 141 and the second resistance voltage divider 142 may have the same impedance under the excitation of the first AC excitation source 121 and the second AC excitation source 122. In some embodiments, by ensuring the symmetry of the first path and the second path, it is possible to make a difference between the detection signal corresponding to the first electrode 111 and the detection signal corresponding to the second electrode 112 originating mainly from a difference in contact impedances between the first electrode 111 and the second electrode 112 and the human body. In this way, by comparing the difference between the detection signals, it is reflected whether fitting states between each of the two electrodes and the human body are good, which in turn reflects whether EMG signals collected by the two electrodes meet a requirement.

Since a frequency of an EMG signal is mainly concentrated in a range of 20 Hz to 400 Hz, and a lower frequency band has Motion Artifacts (MA) with higher intensity, so to avoid being affected by the EMG signal and MA. In some embodiments, a frequency of the AC excitation source (e.g., the first AC excitation source 121 and the second AC excitation source 122) is greater than 250 Hz. Since there is also interference from industrial frequency (e.g., AC power supply of frequency of 50 Hz or 60 Hz AC and harmonics thereof) noise in the signal measurement circuit 100, in some embodiments, the frequency of the AC excitation source may also avoid a frequency range in which the industrial frequency noise is located. In some embodiments, the frequency of the AC excitation source should be at least 1 Hz different from any integer multiple of 50 Hz or 60 Hz, or the frequency of the AC excitation source should be at least 2% different from any integer multiple of 50 Hz or 60 Hz. In some embodiments, the frequency of the AC excitation source is 400 Hz or more, such as 460 Hz, 640 Hz, 830 Hz, or the like. In some embodiments, the setting of the frequency of the AC excitation source also needs to be considered in conjunction with a sampling frequency of the processing circuit 130. Specifically, in order to collect the detection signal, the sampling frequency is at least two times the frequency of the AC excitation source and more. In order to minimize errors, in some embodiments, the sampling frequency is four times and more than the frequency of the AC excitation source. It should be noted that, since a count of channels to be sampled is high and most of the processing circuits 130 have difficulty in supporting a very high sampling frequency of multiple channels, a sampling frequency of the processing circuit 130 and the frequency of the AC excitation source also should not be too high. Based on this, in some embodiments, the frequency of the AC excitation source is set in a range of 250Hz to 2000Hz.

For example, in some embodiments, frequencies of the first AC excitation source 121 and the second AC excitation source 122 are in a range of 500Hz to 1800Hz. In some embodiments, the frequencies of the first AC excitation source 121 and the second AC excitation source 122 is in a range of 450Hz to 2000Hz. In some embodiments, the frequencies of the first AC excitation source 121 and the second AC excitation source 122 is in a range of 600Hz to 1600Hz. In some embodiments, the frequencies of the first AC excitation source 121 and the second AC excitation source 122 is in a range of 850Hz to 1000Hz.

It should be noted that the enumerated values or ranges of the frequencies of the first AC excitation source 121 and the second AC excitation source 122 are only exemplary illustrations, and in some other embodiments, the first AC excitation source 121 and the second AC excitation source 122 may have frequencies at values or ranges other than the enumerated values above. In some embodiments, the frequencies of the first AC excitation source 121 and the second AC excitation source 122 may not be same. For example, the frequencies of the first AC excitation source 121 and the second AC excitation source 122 may have a certain difference, which is within a certain range, such as less than 5% of the frequency of either of the first AC excitation source 121 and the second AC excitation source 122.

In some embodiments, the first resistance voltage divider 141 and the second resistance voltage divider 142 may include a capacitor and/or a resistor. The first resistance voltage divider 141 and the second resistance voltage divider 142 may have different impedances at different frequencies. For example, the first resistance voltage divider 141 and the second resistance voltage divider 142 may present a less impedance when the frequency of the excitation signal is higher, and when the frequency of the excitation signal is lower, the first resistance voltage divider 141 and the second resistance voltage divider 142 may present a higher impedance. Through such arrangement, at the frequency of the excitation signal (e.g., a higher frequency), the first resistance voltage divider 141 and the second resistance voltage divider 142 have less impedances, thereby resulting in less voltage division of the excitation signal, so that the detection signal may more reflect the contact impedance between each of the electrodes and the human body. However, at a lower frequency corresponding to most of the EMG signals, the first resistance voltage divider 141 and the second resistance voltage divider 142 have greater impedances, which avoids the reduction of an input impedance of the subsequent processing circuit 130 due to a parallel connection, so that the EMG signal is picked up and processed by a subsequent processing circuit more effectively. In some embodiments, a general selection of suitable resistors, capacitors, or combinations thereof is made such that a ratio of impedances presented by the first resistance voltage divider 141 and the second resistance voltage divider 142 under the excitation at the frequency of the AC excitation source (e.g., in a range of 250 Hz to 1,000 Hz) to impedances presented by the first resistance voltage divider 141 and the second resistance voltage divider 142 at a frequency of less than 400 Hz is not greater than 1.6. Further, in some embodiments, it is possible to make a ratio of the impedances presented by the first resistance voltage divider 141 and the second resistance voltage divider 142 under the excitation at the frequency of the AC excitation source (e.g., in a range of 250 Hz to 1000 Hz) to impedances presented by the first resistance voltage divider 141 and the second resistance voltage divider 142 under the excitation at a frequency of 50 Hz not greater than 0.2. A specific description of the resistance voltage divider can be found in other parts of the present disclosure, for example, FIG. 3 and its description.

FIG. 3 is a schematic diagram illustrating a signal measurement circuit according to some other embodiments of the present disclosure.

Referring to FIG. 3, in some embodiments, the first AC excitation source 121 (i.e., an AC excitation source connected to a resistor Ra), the first resistance voltage divider 141 (i.e., a resistor Ra), the first electrode 111, a contact impedance Rc, an impedance Re, and a reference ground electrode 113 may constitute a first path. The second AC excitation source 122 (i.e., an AC excitation source connected to a resistor Rb), the second resistance voltage divider 142 (i.e., a resistor Rb), the second electrode 112, a contact impedance Rd, an impedance Re, and the reference ground electrode 113 may form a second path. The contact impedance Rc refers to a contact impedance between the first electrode 111 and a human body, the contact impedance Rd refers to a contact impedance between the second electrode 112 and the human body, and the impedance Re refers to an impedance between the reference ground electrode 113 and the human body. In some embodiments, the impedance Re may include the contact impedance between the reference ground electrode 113 and the human body, as well as an impedance of the human body itself.

Referring to FIG. 3, in some embodiments, when resistance values (Ra and Rb) of the first resistance voltage divider 141 and the second resistance voltage divider 142 are too small, an input impedance of the subsequent processing circuit 130 is too small due to the parallel connection, which results in an intensity of a collected EMG signal being too small and difficult to be monitored. For example, the EMG signal is likely to be overwhelmed by a variety of noises (including aliasing noises, thermal noises, etc.), which will result in a system cannot operate normally. And, the input impedance of the processing circuit 130 being too small also tends to weaken the suppression ability of the input impedance against the industrial frequency noise, thus causing the EMG signal and/or a detection signal to be interfered by the industrial frequency noise. When the resistance values of the first resistance voltage divider 141 and the second resistance voltage divider 142 are too large, this will result in a too large voltage-dividing of an excitation signal, making an intensity of the detection signal too small to reflect a contact impedance between an electrode and the human body.

Considering the above, in some embodiments of the present disclosure, a capacitor is used as a resistance voltage divider. In other words, i.e., in some embodiments of the present disclosure, the first resistance voltage divider 141 and the second resistance voltage divider 142 may employ capacitive elements. Taking a resistance voltage divider of a capacitor with 3pF as an example, it exhibits an impedance of 1.06GS2 at 50Hz (a power frequency), which can effectively suppress the noise at the power frequency, and for about 1000Hz (a preferred frequency of the excitation source), the resistance voltage divider exhibits an impedance of 53MS2, and even for a fluctuation of a contact impedance of 1KS2, a detection signal of 11.3mV is obtained.

In some embodiments, when the first resistance voltage divider 141 and the second resistance voltage divider 142 employ a capacitive element, in order to minimize the effect on the EMG signal, capacitance values corresponding to the first resistance voltage divider 141 and the second resistance voltage divider 142 may need to be controlled at 50 pF and below. At the same time, considering that the less capacitance values corresponding to the first resistance voltage divider 141 and the second resistance voltage divider 142 may make the precision of the detection signal lower, in order to ensure the voltage-dividing precision of the first resistance voltage divider 141 and the second resistance voltage divider 142, the capacitance values corresponding to the first resistance voltage divider 141 and the second resistance voltage divider 142 may also be controlled to be 1 pF and above.

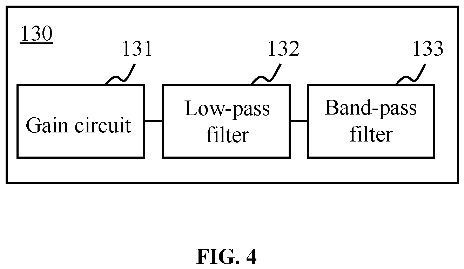

FIG. 4 is a schematic diagram illustrating a processing circuit according to some embodiments of the present disclosure.

Referring to FIG. 3 and FIG. 4, in some embodiments, the processing circuit 130 may include a gain circuit 131 that may take detection signals corresponding to the first electrode 111 and the second electrode 112 as inputs for differential amplification. Since a first path and a second path may remain symmetrical, in some embodiments, a difference between the detection signal corresponding to the first electrode 111 and the detection signal corresponding to the second electrode 112 may reflect a difference in contact impedances between the first electrode 111 and the second electrode 112 and a human body. That is to say, the gain circuit 131 may output the difference between the detection signals corresponding to the difference in contact impedances. In some embodiments, the gain circuit 131 may also simultaneously take a physiological signal (e.g., an EMG signal) as an input and provide gain to the physiological signal. Subsequently, an analog filter, a digital filter, or a signal separation algorithm is utilized to extract an amplified physiological signal and an amplified detection signal, respectively.

In some embodiments, the gain circuit 131 may include a differential amplification circuit, specifically, as shown in FIG. 3. An input end of the gain circuit 131 is connected to the first electrode 111 and the second electrode 112, respectively. Through the gain circuit 131, the difference between the detection signals corresponding to the first electrode 111 and the detection signal corresponding to the second electrode 112 is amplified.

It should be noted that in some embodiments of the present disclosure, the gain circuit 131 may include, but is not limited to, the differential amplification circuit as described previously. For example, in some embodiments, the gain circuit 131 may also include a current amplification circuit, a voltage amplification circuit, a low-frequency amplification circuit, a high-frequency amplification circuit, a logarithmic amplification circuit, an exponential amplification circuit, or the like. In some embodiments, the processing circuit 130 may include other circuits in addition to the gain circuit 131. For example, referring to FIG. 3, the processing circuit 130 may also include an analog-to-digital converter (ADC) disposed at the output end of the gain circuit 131, which is configured to perform an analog-to-digital conversion on the physiological signal and the detection signals to generate corresponding digital signals.

Referring to FIG. 4, in some embodiments, the processing circuit 130 may further include a low-pass filter 132 and a band-pass filter 133 disposed at an output end of the analog-to-digital converter (ADC). The low-pass filter 132 is configured to extract the physiological signal that that is analog-to-digital converted by the analog-to-digital converter, and the band-pass filter 133 is configured to extract the detection signals that are analog-to-digital converted by the analog-to-digital converter and is subjected to differential amplification. As previously mentioned, an output of the band-pass filter 133 is configured to reflect contact impedance information between the electrode and the human body.

In some embodiments, a cutoff frequency of the low-pass filter 132 is determined based on a frequency of an EMG signal. In some embodiments, the cutoff frequency of the low-pass filter 132 is set to 400 Hz, by which the low-pass filter 132 may extract an EMG signal whose frequency is less than 400 Hz. A band-pass frequency range of the band-pass filter 133 is correlated with a frequency of an excitation signal provided by an AC excitation source. For example, when the frequency of the excitation signal provided by the AC excitation source is 1000 Hz, the band-pass frequency range of the band-pass filter 133 is in a range of 990 Hz to 1.01 KHz. A detection signal generated by the AC excitation source reflecting the contact impedance information is primarily extracted by the band-pass filter 133. Furthermore, for example, when the frequency of the excitation signal provided by the AC excitation source is 900 Hz, an extraction threshold range of the band-pass filter 133 is in a range of 890 Hz to 910 Hz. By analogy, when the excitation signal provided by the AC excitation source is other frequencies, the band-pass frequency range of the band-pass filter 133 may also be different ranges.

In some embodiments, the band-pass filter 133 is replaced with a high-pass filter. Similarly, a cutoff frequency of the high-pass filter is correlated with the frequency of the excitation signal provided by the AC excitation source. For example, the cutoff frequency of the high-pass filter should be less than the frequency of the excitation signal provided by the AC excitation source. In some embodiments, in the presence of a plurality of excitation signals at different frequencies, a plurality of band-pass filters 133 or a plurality of high-pass filters is utilized to extract a plurality of detection signals at different frequencies. Cutoff frequencies of the plurality of band-pass filters 133 or the plurality of high-pass filters are different, and a cutoff frequency of each of the plurality of filters is determined based on a frequency of one of a plurality of excitation signals.

In some embodiments, there is a large distance between the first electrode 111 and the second electrode 112 (e.g., greater than or equal to 5 cm) such that the first path and the second path may pass through different body tissues. In some embodiments, the AC excitation source may provide excitation signals of different frequencies for the electrodes (e.g., the first electrode 111 and the second electrode 112) to collect different detection signals. For example, the AC excitation source is further configured to provide a second excitation signal at a different frequency than the excitation signal to generate a second detection signal. The second detection signal may reflect a contact impedance between each of the two electrodes and the human body at the other frequency and the human body's impedance itself. In this way, by combining the contact impedances between each of the electrodes and the human body at different frequencies as well as the human body's impedance at different frequencies, the response of the human body's skin, body composition, or the like to electrical signals of different frequencies is recognized, then body composition information of the human body is determined.

In some embodiments, the AC excitation source may generate excitation signals at different frequencies, respectively, during a plurality of signal collections. Exemplarily, during a first signal collection, the first AC excitation source 121 and the second AC excitation source 122 are configured to provide the first electrode 111 and the second electrode 112 with an excitation signal with a first frequency, so as to generate a first detection signal for reflecting a contact impedance between each of the two electrodes and the human body at that first frequency, and subsequently, during a second signal collection, the first AC excitation source 121 and the second AC excitation source 122 are configured to provide the first electrode 111 and the second electrode 112 with an excitation signal having a second frequency (e.g., the second excitation signal) for generating a second detection signal for reflecting a contact impedance between each of the two electrodes and the human body at the second frequency (e.g., the other frequency as previously described). The first frequency and the second frequency may be different. In some embodiments, the first frequency and the second frequency are in a range of 250Hz to 1000Hz.

It should be noted that the AC excitation source may provide excitation signals of different frequencies for the electrodes during different processes of signal collection. Or the AC excitation source may provide excitation signals of the same frequency for the electrodes during a plurality of processes of signal collection, and the AC excitation source may provide excitation signals of different frequencies for the electrodes during a same process of signal collection.

In some embodiments, the AC excitation source may also generate excitation signals at a plurality of frequencies simultaneously. Exemplarily, the first AC excitation source 121 and the second AC excitation source 122 provide the first electrode 111 and the second electrode 112 with both an excitation signal having the first frequency and an excitation signal having the second frequency to simultaneously generate a first detection signal at the first frequency and a second detection signal at the second frequency. In some embodiments, the AC excitation source may also generate a composite excitation signal, which may have a plurality of frequencies. Exemplarily, the AC excitation source provides both the first electrode 111 and the second electrode 112 with a composite excitation signal having the first frequency and the second frequency to simultaneously generate the first detection signal at the first frequency and the second detection signal at the second frequency. In some embodiments, a filter is utilized to extract the first detection signal and the second detection signal, respectively, based on different frequencies of the first detection signal and the second detection signal.

In some embodiments, the processing circuit 130 evaluates the body composition information of the human body based on the first detection signal and the second detection signal. For example, the first detection signal and the second detection signal are weighted and averaged so as to determine the body composition information such as a body fat rate, a bone density, a body fluid content, or the like of the human body. Furthermore, for example, the response of the human body's impedance to electrical signals of different frequencies is used as an input, and a machine learning model is configured to output a result that reflects the body composition information.

In some embodiments, the AC excitation source generates two or more excitation signals having different frequencies, such as a first excitation signal at a first frequency, a second excitation signal at a second frequency, a third excitation signal at a third frequency, or the like. The third frequency is different from both the first frequency and the second frequency as described previously. Further, the processing circuit 130 may evaluate the body composition information of the human body based on the first detection signal, the second detection signal, and a third detection signal. It is to be noted that the foregoing enumeration of the first excitation signal, the second excitation signal, and the third excitation signal is only an exemplary illustration, and excitation signals generated by the AC excitation source may include other signals in addition to those enumerated above.

It should be noted that the foregoing description of the signal measurement circuit 100 is provided for illustrative purposes only and is not intended to limit the scope of the present disclosure. It is to be understood that it is possible for a person skilled in the art to deform the signal measurement circuit 100 according to the description of the present disclosure, possibly without departing from this principle. For example, in some embodiments, the first electrode 111 and the second electrode 112 are connected to different processing circuits 130, respectively. Furthermore, for example, in some embodiments, the signal measurement circuit 100 includes a resistance voltage divider other than the first resistance voltage divider 141 and the second resistance voltage divider 142. Deformations such as these are within the scope of protection of the present disclosure.

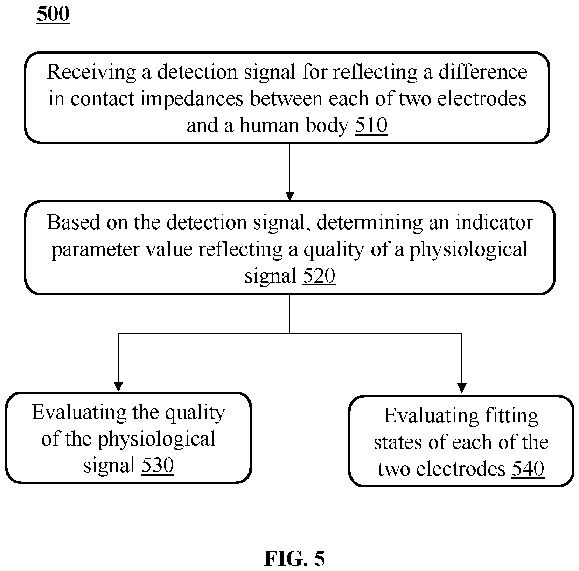

FIG. 5 is a flowchart illustrating a signal measurement method according to some embodiments of the present disclosure. In some embodiments, a process 500 is executed by a smart wearable device as described in the foregoing, which may include a processor, a memory, and the signal measurement circuit 100. The memory may store a computer program for executing the process 500, and the processor is a part of the processing circuit 130 in the signal measurement circuit 100, or is an independently provided electronic device capable of implementing functions of the processing circuit 130. Further details on the signal measurement circuit 100 can be referred to FIG. 1 to FIG. 4 and their related discussions.

Referring to FIG. 5, in some embodiments, the process 500 may include:
Step 510, a detection signal for reflecting a difference in contact impedances between each of two electrodes and a human body is received. In some embodiments, step 510 is implemented by electrodes in the signal measurement circuit 100.

According to the above description of the signal measurement circuit 100, in some embodiments, two electrodes (e.g., the first electrode 111 and the second electrode 112) in the signal measurement circuit 100 is configured to affix to the human body to collect a physiological signal (e.g., an EMG signal) of the human body. In the case where an AC excitation source is provided, a detection signal for reflecting a contact impedance between each of the two electrodes and the human body may also be collected simultaneously. In some embodiments, the detection signal is obtained by calculating a difference between detection signals obtained from different electrodes, and the detection signal may reflect a difference in contact impedances between each of the two electrodes and the human body.

Since the detection signal is generated by an excitation signal provided by the AC excitation source, a frequency of the detection signal is different from a frequency of the physiological signal. In some embodiments, the detection signal corresponding to the electrode is obtained by filtering a collected signal. For example, when the frequency of the detection signal is 1000 Hz, the detection signal or the detection signal is obtained by extracting the collected signal utilizing a band-pass filter of 990 Hz to 1.01 KHz. In some embodiments, the detection signal is a sine wave or a cosine wave having the same frequency as the excitation signal provided by the AC excitation source, and a higher magnitude indicates a higher contact impedance between the electrode and the human skin.

Step 520, based on the detection signal, an indicator parameter value reflecting the quality of the physiological signal is determined. In some embodiments, step 520 is realized by the processing circuit 130 in the signal measurement circuit 100.

To aid in determining a correspondence between the quality of the physiological signal and the detection signal, in some embodiments, the indicator parameter value reflecting the quality of the physiological signal is determined based on the detection signal. In some embodiments, the indicator parameter value is generated based on a digitally sampled value of the detection signal, which is configured to reflect the quality of the physiological signal. In some embodiments, for a detection signal within a certain period, the detection signal is sampled in segments, for example, the detection signal is sampled a plurality of times using a preset sampling duration, and based on a feature parameter of each sampling window, an indicator parameter value corresponding to each period is determined. For example, the detection signal within a certain period is processed using the de-negative (abs) function to obtain a digital absolute value of the detection signal within the period. A feature parameter (e.g., an average magnitude) of a detection signal within a first preset sampling period is taken as a first sampling value, and then cyclic sampling is carried out at intervals of a specific period, so as to obtain an indicator parameter value corresponding to a detection signal within a period.

In some embodiments, the indicator parameter value may reflect a difference in contact impedances between each of the two electrodes and the human body, thereby reflecting a difference in fitting states between each of the two electrodes and the human body, thereby reflecting the quality of a corresponding physiological signal.

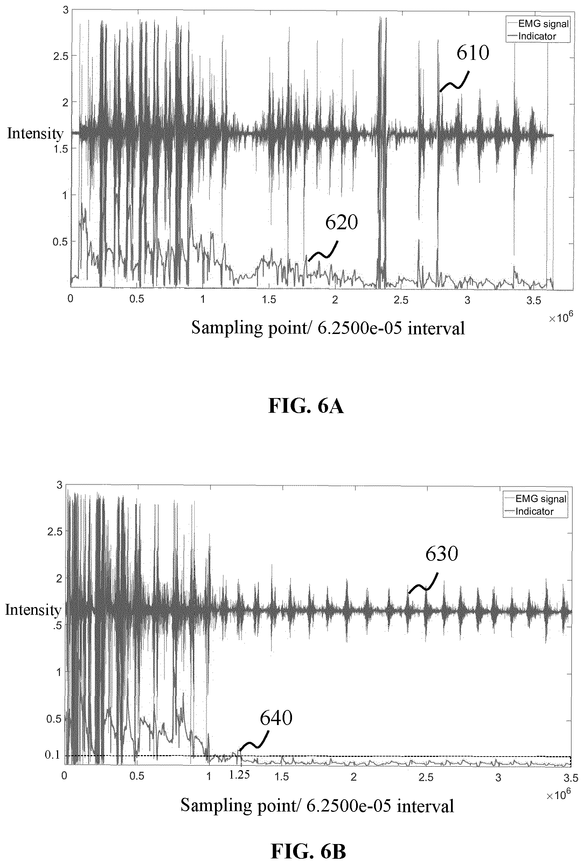

In some embodiments, the detection signal is periodic, and the indicator parameter value is a representation of a detection signal corresponding to at least one cycle. In some embodiments, the indicator parameter value may include a magnitude value of the detection signal at a preset moment and/or a fluctuation of magnitude values of the detection signal within a preset period. For example, in some embodiments, the indicator parameter value is a magnitude value corresponding to a detection signal at 1/2 cycle. In some embodiments, the indicator parameter value is a maximum value, a minimal value, or an average value of a detection signal with a cycle. For a more detailed description of the indicator parameter value, reference is made to the rest of the present disclosure, for example, FIGs. 6A to 6B and related descriptions.

Step 530, the quality of the physiological signal is evaluated. Step 530 is realized by the processing circuit 130 in the signal measurement circuit 100.

In some embodiments, the quality of the physiological signal is evaluated based on the indicator parameter value to determine whether to output the physiological signal. For example, if the quality of the physiological signal is high, the physiological signal is output. If the quality of the physiological signal is poor, the physiological signal is not output.

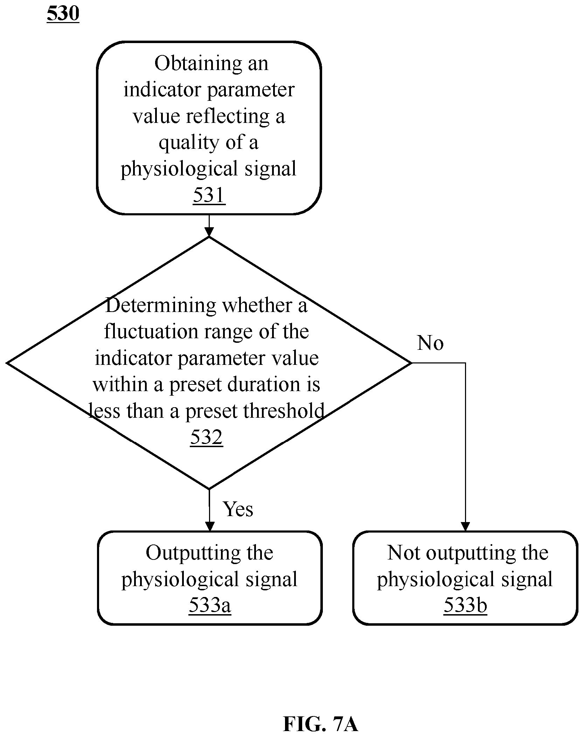

In some embodiments, a processor may also generate quality information for evaluating the quality of the physiological signal based on a fluctuation of magnitude values of the indicator parameter value within a preset duration. Optionally, after generating the quality information, the processor may further determine whether to output the physiological signal based on the quality information. Detailed processes regarding step 530 may be referred to FIG. 7A and its related discussions.

Step 540, a fitting state of each of the two electrodes is evaluated. Step 540 may be realized by the processing circuit 130 in the signal measurement circuit 100.

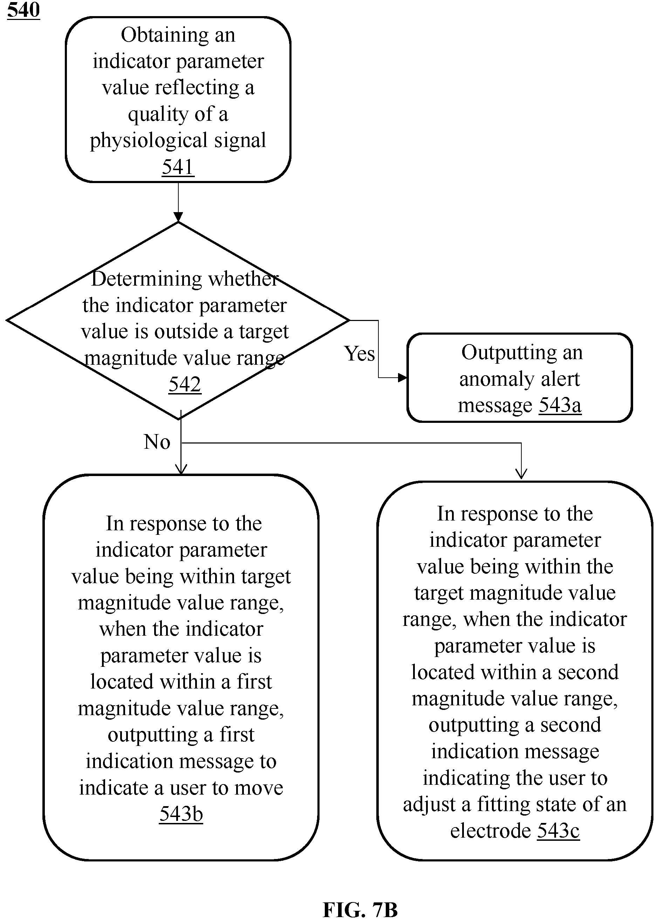

Since the indicator parameter value reflects the difference in contact impedances between each of the two electrodes and the human body, a large difference in contact impedances (e.g., when a user performs small-amplitude exercise or when the user's skin is dry) may indicate that both electrodes have a large contact impedance, and an approximate absolute value of each contact impedance is obtained by speculation. In some embodiments, the contact impedance is evaluated based on the indicator parameter value. In some embodiments, the processor may also generate parameter information for evaluating the fitting states of the electrodes based on the indicator parameter value. In some embodiments, the parameter information may include impedance information between each of the two electrodes and the human body, by which the fitting state between each of the electrodes and the human body is reflected, which in turn can reflect a current wear state of the wearable device and a size fit, or the like. Detailed procedures regarding step 540 can be referred to FIG. 7B and its related discussions.

The following two exemplary signal measurement results are provided separately to describe in detail how the indicator parameter value is implemented.

FIG. 6A and FIG. 6B are waveform diagrams of signal measurement results according to some embodiments of the present disclosure. Referring to FIG. 6A and FIG. 6B, a physiological signal curve 610 represents an EMG signal of a pectoral muscle region during a first movement, and indicator parameter value curve 620 represents an indicator parameter value Indicator during the first movement. A physiological signal curve 630 represents an EMG signal of the pectoral muscle region during a second movement, and an indicator parameter value curve 640 represents an indicator parameter value Indicator during the second movement.

According to FIG. 6A and FIG. 6B, it can be seen that when the physiological signal curve 610 (the EMG signal in the present disclosure) generates a large fluctuation, a fluctuation of the indicator parameter value curve 620 (Indicator) is also large (as shown in FIG. 6A), and when the physiological signal curve 630 (the EMG signal in the present disclosure) is flatter, the indicator parameter value curve 640 (Indicator) also decreases to a certain extent (as shown in FIG. 6B). The indicator parameter value Indicator increases significantly when noise (e.g., burrs appearing in a waveform diagram) is present in the physiological signal curve 610. Thereby, the indicator parameter value provided by embodiments of the present disclosure may reflect the quality of a physiological signal.

In some embodiments, a fitting state of an electrode is evaluated based on a comparison result between the indicator parameter value and a preset indicator threshold. Exemplarily, if when the indicator parameter value Indicator is <0.1 (e.g., the indicator parameter value curve 640 is within a sampling point interval 1.25*10^6-3.5*10^6), it is indicated that a contact impedance between the electrode and a human body is small. Thus, a fitting state between the electrode and the human body is good. When the indicator parameter value Indicator>0.1, it indicates that the contact impedance between the electrode and the human body is large. Thus, the fitting state between the electrode and the human body is poor. In some embodiments, the fitting state between the electrode and the human body may include one or more pieces of following information: a current wear state of a wearable device, a size fit, a warm-up state, and a skin state where the electrode contacts with the human body. When the fitting state between the electrode and the human body is good, it may indicate that the current wear state of the wearable device is good, that the size of the wearable device is appropriate, and that a user is in a sufficiently warmed-up state, and that the skin where the electrode contacts with the human body is in a moister state. A poor fitting state between the electrode and the human body may indicate one or a combination of the following: the current wear state of the wearable device is poor, the size of the wearable device does not fit, the user is not fully warmed up, the skin where the electrode contacts with the human body is dry.

Further, in some embodiments, the current wearing state of the wearable device, the size fit, the warm-up state, the skin state where the electrode contacts with the human body, or other wearing information may also be evaluated based on a comparison relationship between the indicator parameter value Indicator and the preset indicator threshold. Exemplarily, when the indicator parameter value Indicator is <0.1, it is determined that the current wearing state of the wearable device is well worn, and it is determined that the size fit of the wearable device is fit, and it may also be determined that the user is in a sufficiently warmed-up state, and the skin where the electrode contacts with the human body skin in a moister state.

Referring to FIG. 6B, at a time when an absolute value of the indicator parameter value Indicator is low (e.g. when a magnitude value of the indicator parameter value curve 640 is below the preset indicator threshold), a movement action performed by the user may also cause an increase in the magnitude value of the indicator parameter value Indicator (e.g., the indicator parameter value curve 640 includes a plurality of peaks within the sampling point interval of 1.25*10^6-3.5*10^6). Based on this, in some embodiments, the movement action of the user is monitored based on a fluctuation of magnitude values of the indicator parameter value within a preset period. Exemplarily, in the case where the indicator parameter value Indicator is <0.1 and the indicator parameter value Indicator includes more than a preset count of peaks within the preset period, it is determined that the user performs a movement action within the preset period.

Since there is a correspondence between a detection signal and the contact impedance between the electrode and the human body, in some embodiments, there is also a correspondence relationship between the indicator parameter value Indicator and the contact impedance between the electrode and the human body. In some embodiments, the fluctuation range of the indicator parameter value Indicator within the preset period reflects changes in the contact impedance within the preset period, thereby reflecting more accurately the quality of the EMG signal. Based on this, in some embodiments, the fluctuation of magnitude values of the indicator parameter value within the preset period (i.e., changes in the magnitude value of the indicator parameter value curve 620 in FIG. 6A) is configured to indicate the quality of the EMG signal. For example, based on a difference between a maximum value and a minimum value of a magnitude value of an indicator parameter value corresponding to a detection signal during an action cycle, a fluctuation of magnitude values ΔIndicator of the detection signal during the action cycle is determined if the fluctuation of magnitude values ΔIndicator of the detection signal during the action cycle is <0.1, it is indicated that it has a high-quality grade for a reflected physiological signal. Similarly, in some embodiments, a relatively next lower quality grade is determined based on the fluctuation of the magnitude value of the indicator parameter value, or the like.

In order to evaluate the quality of the physiological signal, in some embodiments, the process 500 may further include: when the indicator parameter value satisfies a preset indicator condition, a physiological signal collected by the electrode is output, for example, by displaying the physiological signal or broadcasting the physiological signal by voice. It is noted that in some embodiments of the present disclosure, an output process of outputting the physiological signal is performed by a smart wearable device comprising the signal measurement circuit 100, or by other terminals or output devices (e.g., a speaker, a display, or the like) communicatively connected to the smart wearable device.

In some embodiments, the preset indicator condition is that a fluctuation range of the indicator parameter value within a preset duration is less than a preset threshold. An amplitude of the preset threshold is set according to a processing algorithm for generating the indicator parameter value. For example, the preset indicator condition is set as that a fluctuation of magnitude values of the indicator parameter value within one action cycle is less than or equal to 0.3. Or the preset indicator condition is set as that a fluctuation of magnitude values of the indicator parameter value within half an action cycle is less than or equal to 0.3.

In some embodiments, when the indicator parameter value does not satisfy the preset indicator condition, then it indicates that there is an anomaly in a detection signal collected by the electrode, and at this time, the physiological signal may not be output.

In some embodiments, the process 500 may further include: when the indicator parameter value does not satisfy the preset indicator condition, feedback information including the indicator parameter value is determined. Then based on the feedback information, a signal processing algorithm is invoked, and the physiological signal is processed to determine a target physiological signal. In some embodiments, the signal processing algorithm may correct or compensate for the physiological signal, thereby ensuring the quality or accuracy of the physiological signal. In some embodiments, the signal processing algorithm may further include at least one of: a signal filtering algorithm, a wavelet transform algorithm, or a machine learning algorithm. In some embodiments, the feedback information is configured to reflect correction or compensation that needs to be made for the physiological signal for subsequent invoke of a signal processing algorithm appropriate for the physiological signal. In some embodiments, the target physiological signal is a physiological signal after correction or compensation. The target physiological signal is of better quality or accuracy compared to the physiological signal. In some embodiments, the target physiological signal is utilized instead of the physiological signal for output or display.

In some embodiments, invoking the signal processing algorithm based on the feedback information may include: determining a weight of the physiological signal based on the indicator parameter value of the feedback information, and invoking, based on a threshold range in which the weight is located, a signal processing algorithm corresponding to the threshold range. In some embodiments, the weight is configured to measure a degree of trustworthiness of the physiological signal in terms of quality. In some embodiments, the weight and the indicator parameter value may have a correspondence relationship. For example, the greater the indicator parameter value, the lower the weight, and the less the indicator parameter value, the higher the weight. In some embodiments, the weight may also be used as a label for the physiological signal, and a machine learning model is trained using the weight and the physiological signal. In some embodiments, the weight of the physiological signal is assigned using a trained machine learning model. In some embodiments, the threshold range corresponding to the weight may have a correspondence relationship with one or more signal processing algorithms, such that the one or more algorithms are invoked to perform a signal processing on the physiological signal.

In some embodiments, invoking the signal processing algorithm based on the feedback information may include: taking the feedback information as an input, running the trained machine learning model, and invoking the signal processing algorithm corresponding to the feedback information. In some embodiments, the machine learning model is trained by using sample feedback information as a label for a sample signal processing algorithm, thereby obtaining the trained machine learning model capable of invoking the signal processing algorithm. It should be noted that the signal processing algorithm may also be invoked in other ways such as by reference to historical feedback information, big data statistics, and so forth, which will not be repeated herein.

In some embodiments, the process 500 may further include: when the indicator parameter value does not satisfy the preset indicator condition, obtaining a historical physiological signal of the human body, and then, based on the historical physiological signal and a currently-collected physiological signal, displaying an alternative physiological signal and/or outputting the alternative physiological signal. In some embodiments, the alternative physiological signal is a desired signal at a current moment derived based on the historical physiological signal and the currently-collected physiological signal. In some embodiments, the alternative physiological signal is obtained by calculating a weighted average of the historical physiological signal and the currently-collected physiological signal. An exemplary process for evaluating the physiological signal is provided below, with detailed specific implementations of evaluating the physiological signal.

FIG. 7A is a flowchart illustrating a signal measurement method according to some other embodiments of the present disclosure.

Referring to FIG. 7A, in some embodiments, step 530 may include:
Step 531, an indicator parameter value reflecting a quality of a physiological signal is obtained.

It is noted that the indicator parameter value in step 531 is an indicator parameter value determined in step 520. In some embodiments, the indicator parameter value may include a magnitude value of a detection signal at a preset moment and/or a fluctuation of magnitude values of the detection signal within a preset period.

Step 532, whether a fluctuation range of the indicator parameter value within a preset duration is less than a preset threshold is determined.

In some embodiments, the fluctuation range of the indicator parameter value within the preset duration is a difference between a maximum magnitude value and a minimum magnitude value of the indicator parameter value within the preset period. In some embodiments, the preset duration is, but is not limited to, one action cycle, half an action cycle, and two action cycles. In some embodiments, the preset threshold is 0.5, 0.4, 0.1, etc., and a specific preset threshold is set based on a fluctuation of a desired indicator parameter value.

Step 533a, the physiological signal when the fluctuation range of the indicator parameter value within the preset duration is less than the preset threshold is output. At this time, the indicator parameter value is in a stable state within the preset duration, indicating that a fitting state of an electrode to a human body is in a stable and good state, and the physiological signal collected by the electrode is considered valid and be further output to a user.

Step 533b, when the fluctuation range of the indicator parameter value within the preset duration is not less than the preset threshold, the physiological signal is not output. At this time, the indicator parameter value is in an unstable state within the preset duration, indicating that the fitting state of the electrode to the human body is in an unstable and poor state, then the physiological signal collected by the electrode is considered invalid and may not be output to the user.

In an embodiment of the present disclosure, based on the fluctuation range of the indicator parameter value within the preset duration to determine whether the physiological signal is needed to be output, it is possible to control the quality of an output physiological signal, and to avoid that physiological signals of poor quality are output to occupy resources. In addition, the fluctuation range of the indicator parameter value within the preset duration may also reflect the user's warm-up state, skin condition, and other information, thus helping the user to accurately understand his or her physical state. It should be noted that the above description of the preset duration and the preset threshold is only an illustrative example. In embodiments of the present disclosure, the preset duration and the preset threshold are, but are not limited to, the above enumerated values.

An exemplary process for evaluating the physiological signal is provided below, and a detailed and specific implementation of evaluating the fitting state of the electrode can be found in the following descriptions.

FIG. 7B is a flowchart illustrating a signal measurement method according to some other embodiments of the present disclosure.

Referring to FIG. 7B, in some embodiments, step 540 may include:
Step 541, an indicator parameter value reflecting a quality of a physiological signal is obtained.

Similar to step 531, the indicator parameter value in step 541 may also be an indicator parameter value determined in step 520. In some embodiments, the indicator parameter value may include a magnitude value of a detection signal at a preset moment and/or a fluctuation of a magnitude value of the detection signal within a preset period.

Step 542, whether the indicator parameter value is outside a target magnitude value range is determined.

In some embodiments, the target magnitude value range is obtained based on historical experience or statistical data. In some embodiments, the target magnitude value range may also be set based on a processing algorithm for generating the indicator parameter value. Exemplarily, the target magnitude value range is in a range of 0 to 1.

Step 543a, an anomaly alert message when the indicator parameter value is located outside the target magnitude value range is output.

When the indicator parameter value is located outside the target magnitude value range, it can reflect that the quality of a collected physiological signal is poor, and it is subsequently difficult to use the physiological signal for physiological monitoring, thus the anomaly alert message needs to be output to remind a user of the timely checking and adjustment. In some embodiments, there are many factors that cause the quality of the physiological signal to be very poor, such as an electrode being detached from the human skin or the user's body being diseased, and so on, and the user may check the factors in time based on the anomaly alert message. In some embodiments, when step 542 determines that the indicator parameter value is located outside the target magnitude value range, a corresponding alert message is output by an output device to remind the user, such as a sound and light prompt, output text /image display, outputting voice, etc.

Step 543b, in response to the indicator parameter value being within the target magnitude value range, when the indicator parameter value is located within a first magnitude value range, a first indication message to indicate the user to move is output.

In some embodiments, when the step 542 determines that the indicator parameter value is located within the target magnitude value range, it may further determine a range to which the indicator parameter value belongs. If the indicator parameter value is located within the first magnitude value range, the first indication message indicating the user to move is output. Exemplarily, the first magnitude value range is in a range of 0.5 to 1. When the indicator parameter value lies within the first magnitude value range, it is indicated that the quality of the physiological signal is still poor, and it is difficult to accurately reflect a physiological state of the user, and thus it is recommended that the user to detect or adjust to improve the quality of the physiological signal. In some embodiments, the user may exercise in accordance with the first indication message to reduce a contact impedance by warming up, thereby improving the quality of the physiological signal. In some embodiments, the first indication message may instruct the user via text, image, voice, or video to properly exercise to reduce an amplitude of the contact impedance, thereby improving the quality of the physiological signal.

Step 543c, in response to the indicator parameter value being within the target magnitude value range, when the indicator parameter value is located within a second magnitude value range, a second indication message indicating the user to adjust a fitting state of an electrode is output.

In some embodiments, if the indicator parameter value is within the second magnitude value range, the second indication message indicating that the user to adjust the fitting state of the electrode is output. Exemplarily, the second magnitude value range is in a range of 0.4 to 0.7, and it should be noted that the first magnitude value range and the second magnitude value range may or may not intersect, and no specific limitations are made herein. When the indicator parameter value is located in the second magnitude value range, it is indicated that there is an anomaly between the fitting state of the electrode and the human skin, such as a poor contact or incomplete affixation, etc., which results in the physiological signal of poorer quality and difficulty in accurately reflecting the physiological state of the user. At this time, the second indication message for instructing the user to adjust the fitting state of the electrode is output to change the amplitude of the contact impedance. Similar to the first indication message, in some embodiments, the second indication message may instruct the user by text, image, voice, or video. It is to be noted that in some embodiments, an overlapping portion may exist between the second magnitude value range and the first magnitude value range, and when the indicator parameter value is located in the overlapping portion, the first indication message and the second indication message are output at the same time.

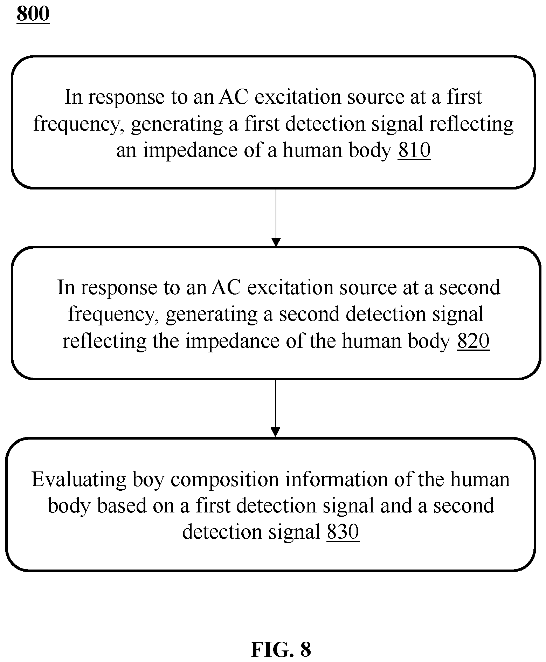

FIG. 8 is a flowchart illustrating a signal measurement method according to some other embodiments of the present disclosure. Similar to the process 500, in some embodiments, a process 800 is performed by a smart wearable device, which may include a processor, a memory, and the signal measurement circuit 100 as described above. The memory may store a computer program for executing the process 800, and the processor is part of the processing circuit 130 in the signal measurement circuit 100. A processor is implemented in a specific manner similar to functions mentioned in FIG. 5 above, and may not be repeated herein.

Referring to FIG. 8, in some embodiments, the process 800 may include:

Step 810, in response to an AC excitation source at a first frequency, a first detection signal reflecting an impedance of a human body is generated.

According to the above description of the signal measurement circuit 100, in some embodiments, the signal measurement circuit 100 may include two or more electrodes. There is a relatively large distance (e.g., greater than or equal to 5 cm) from each other between two electrodes (e.g., the first electrode 111 and the second electrode 112), and an AC excitation source in the signal measurement circuit 100 (e.g., the first AC excitation source 121 and/or the second AC excitation source 122) may have different frequencies, which may generate excitation signals of different frequencies. In some embodiments, when the AC excitation source provides an excitation signal of a first frequency to an electrode, the signal measurement circuit 100 generates a first detection signal reflecting the impedance of the human body (the impedance generated by the tissues within the human body). For example, detection signals formed by the voltage-division of the two or more electrodes under the action of the excitation signal at the first frequency, or a difference between the detection signals is used as the first detection signal of the impedance of the human body at the first frequency. In some embodiments, the impedance of the human body reflected in the first detection signal may include a contact impedance between the electrode and the human skin.

Step 820, in response to an AC excitation source at a second frequency, a second detection signal reflecting the impedance of the human body is generated.

When the AC excitation source provides the electrode with an excitation signal of a second frequency, the signal measurement circuit 100 may generate the second detection signal reflecting the impedance of the human body. In the same manner as the first detection signal, in some embodiments, the second detection signal is obtained by processing detection signals collected, respectively, under the action of the excitation signal of the second frequency. For example, detection signals formed by the voltage-division of the two or more electrodes under the action at the excitation signal of the second frequency, or a difference between the detection signals, is used as the second detection signal of the impedance of the human body at the second frequency.

It should be noted that in some embodiments, detection signals obtained and collected at different frequencies is processed by the same or different machine learning models to obtain the first detection signal and second detection signal.

Step 830, body composition information of the human body is evaluated based on the first detection signal and the second detection signal.

Since differentiated body composition information is obtained at different frequencies, after obtaining the first detection signal and the second detection signal generated at different frequencies, the first detection signal and the second detection signal is processed to evaluate the body composition information. In some embodiments, an evaluation result of the body composition information is obtained by processing through a body composition analysis model. In some embodiments, the body composition analysis model may also be a machine learning model. For example, the first detection signal and the second detection signal are inputted into the body composition analysis model, and a corresponding evaluation result of body composition information is output using the body composition analysis model. In some embodiments, the evaluation result may include parameters such as a body fat rate, a body fat rate, a bone density, a body fluid content, or the like. In some embodiments, each sample may include an evaluation result of body composition information and corresponding detection signals at different frequencies, and a plurality of samples are utilized to train or update the body composition analysis model.

It should be noted that in embodiments of the present disclosure, the foregoing body composition analysis model and a machine learning model for determining the first detection signal and the second detection signal are obtained by training. In some embodiments, the training of the body composition analysis model and the machine learning model for determining the first detection signal and the second detection signal may include supervised learning and/or unsupervised learning.

In some embodiments, after obtaining an evaluation result of the body composition information of a user, the evaluation result is output by an output device. An outputting manner regarding the evaluation result is referred to in the first indication message and the second indication message, and will not be repeated herein.

Beneficial effects that may be brought about by embodiments of the present disclosure include, but are not limited to: (1) by means of the signal measurement method and circuit provided in embodiments of the present disclosure, it is possible to detect in real time the contact impedance between each of the electrodes and the human body, determine the fitting state between each of the electrodes and the human body based on the contact impedance, and thus ensure the quality of the physiological signal collected by the electrodes; (2) in some embodiments of the present disclosure provided in the signal measurement circuit, differentiated detection signals are obtained by using excitation signals of different frequencies and electrodes with different paths, and the body composition information of the human body is obtained after further processing; (3) in some embodiments of the present disclosure, through the contact impedance between each of the electrodes and the human body, it is also possible to further analyze a wearing state of the wearable device, the size fit, the warm-up state, the skin state, or the like.

It should be noted that the beneficial effects that may be produced by different embodiments are different, and the beneficial effects that may be produced in different embodiments may be any one or a combination of any one or a combination of any of the foregoing, or any other beneficial effect that may be obtained.

The basic concepts have been described above, and it is apparent to those skilled in the art that the foregoing detailed disclosure serves only as an example and does not constitute a limitation of the present disclosure. While not expressly stated herein, various modifications, improvements, and amendments may be made to the present disclosure by those skilled in the art. Those types of modifications, improvements, and amendments are suggested in the present disclosure, so those types of modifications, improvements, and amendments remain within the spirit and scope of the exemplary embodiments of the present disclosure.

Also, the present disclosure uses specific words to describe embodiments of the present disclosure. Such as "an embodiment", "one embodiment", and/or "some embodiments" means a feature, structure, or characteristic associated with at least one embodiment of the present disclosure. Accordingly, it should be emphasized and noted that two or more references to "one embodiment", "an embodiment", or "an alternative embodiment" in different places in the present disclosure do not necessarily refer to the same embodiment. In addition, certain features, structures, or characteristics of one or more embodiments of the present disclosure may be suitably combined.

Furthermore, unless expressly stated in the claims, the order of the processing elements and sequences, the use of numerical letters, or the use of other names as described in the present disclosure are not intended to qualify the order of the processes and methods of the present disclosure. While some embodiments of the invention that are currently considered useful are discussed in the foregoing disclosure by way of various examples, it should be appreciated that such details serve only illustrative purposes, and that additional claims are not limited to the disclosed embodiments, rather, the claims are intended to cover all amendments and equivalent combinations that are consistent with the substance and scope of the embodiments of the present disclosure. For example, although the implementation of various components described above may be embodied in a hardware device, it may also be implemented as a software only solution, e.g., an installation on an existing server or mobile device.

Similarly, it should be noted that in order to simplify the presentation of the present disclosure, and thereby aid in the understanding of one or more embodiments of the invention, the foregoing descriptions of embodiments of the present disclosure sometimes group multiple features together in a single embodiment, accompanying drawings, or in a description thereof. However, this method of disclosure does not imply that the objects of the present disclosure require more features than those mentioned in the claims. Rather, claimed subject matter may lie in less than all features of a single foregoing disclosed embodiment.

Some embodiments use numbers describing the number of components, attributes, and it should be understood that such numbers used in the description of embodiments are modified in some examples by the modifiers "approximately", "nearly", or "substantially". Unless otherwise noted, the terms "approximately", "nearly", or "substantially" indicates that a ±20% variation in the stated number is allowed. Correspondingly, in some embodiments, the numerical parameters used in the present disclosure and claims are approximations, which can change depending on the desired characteristics of individual embodiments. In some embodiments, the numerical parameters should take into account the specified number of valid digits and employ general place-keeping. While the numerical domains and parameters configured to confirm the breadth of their ranges in some embodiments of the present disclosure are approximations, in specific embodiments, such values are set to be as precise as possible within a feasible range.

For each of the patents, patent applications, patent application disclosures, and other materials cited in the present disclosure, such as articles, books, specification sheets, publications, documents, or the like, are hereby incorporated by reference in their entirety into the present disclosure. Application history documents that are inconsistent with or conflict with the contents of the present disclosure are excluded, as are documents (currently or hereafter appended to the present disclosure) that limit the broadest scope of the claims of the present disclosure. It should be noted that in the event of any inconsistency or conflict between the descriptions, definitions, and/or use of terms in the materials appended to the present disclosure and those set forth herein, the descriptions, definitions and/or use of terms in the present disclosure shall prevail.

Finally, it should be understood that the embodiments described in the present disclosure are only used to illustrate the principles of the embodiments of the present disclosure. Other deformations may also fall within the scope of the present disclosure. As such, alternative configurations of embodiments of the present disclosure may be viewed as consistent with the teachings of the present disclosure as an example, not as a limitation.
Correspondingly, the embodiments of the present disclosure are not limited to the embodiments expressly presented and described herein.

## Claims

1. A signal measurement method, comprising:
receiving a detection signal, the detection signal reflecting a difference between two contact impedances, each of which is a contact impedance between one of two electrodes and a human body, and the two electrodes being affixed to the human body and being configured to collect a physiological signal of the human body; and
determining an indicator parameter value reflecting a quality of the physiological signal based on the detection signal.

2. The signal measurement method of claim 1, wherein the indicator parameter value includes a magnitude value of the detection signal at a preset moment and/or a fluctuation of magnitude values of the detection signal within a preset period.

3. The signal measurement method of claim 2, wherein the detection signal is periodic, and the indicator parameter value is a representation of the detection signal corresponding to at least one cycle.

4. The signal measurement method of claim 1, further comprising:
in response to determining that the indicator parameter value satisfies a preset indicator condition, displaying the physiological signal, wherein the preset indicator condition is that a fluctuation within a preset duration is less than a preset threshold value.

5. The signal measurement method of claim 4, further comprising: in response to determining that the indicator parameter value does not satisfy the preset indicator condition, not displaying the physiological signal.

6. The signal measurement method of claim 4, further comprising:
in response to determining that the indicator parameter value does not satisfy the preset indicator condition, determining feedback information including the indicator parameter value; and
determining a target physiological signal by invoking a signal processing algorithm based on the feedback information and processing the physiological signal.

7. The signal measurement method of claim 6, wherein the signal processing algorithm includes at least one of: a signal filtering algorithm, a wavelet transform algorithm, or a machine learning algorithm.

8. The signal measurement method of claim 6, wherein the invoking the signal processing algorithm based on the feedback information includes:
determining a weight of the physiological signal based on the indicator parameter value of the feedback information; and
invoking, based on a threshold range in which the weight is located, the signal processing algorithm corresponding to the threshold range.

9. The signal measurement method of claim 6, wherein the invoking the signal processing algorithm based on the feedback information includes:
invoking the signal processing algorithm corresponding to the feedback information by using the feedback information as an input of a trained machine learning model and running the trained machine learning model.

10. The signal measurement method of claim 4, further comprising:
in response to determining that the indicator parameter value does not satisfy the preset indicator condition, obtaining a historical physiological signal of the human body; and
displaying an alternative physiological signal based on the historical physiological signal and the physiological signal.

11. The signal measurement method of claim 4, further comprising:
generating quality information for assessing the quality of the physiological signal based on a fluctuation range of the indicator parameter value within the preset duration.

12. The signal measurement method of claim 1, further comprising:
generating parameter information for evaluating fitting states of the two electrodes based on the indicator parameter value.

13. The signal measurement method of claim 1, further comprising:
in response to determining that the indicator parameter value is not within a target magnitude value range, outputting an anomaly alert message.

14. The signal measurement method of claim 13, wherein the target magnitude value range includes a first magnitude value range, the signal measurement method further comprising:
in response to determining that the indicator parameter value is within the first magnitude value range, outputting a first indication message, the first indication message being configured to indicate a user to move.

15. The signal measurement method of claim 13, wherein the target magnitude value range includes a second magnitude value range, the signal measurement method further comprising:
in response to determining that the indicator parameter value is within the second magnitude value range, outputting a second indication message, the second indication message being configured to instruct a user to adjust fitting states of the two electrodes.

16. A signal measurement circuit, comprising:
two electrodes, configured to affix to a human body to collect a physiological signal of the human body;
an alternating current (AC) excitation source electrically connected to each of the two electrodes, the AC excitation source being configured to provide an excitation signal to generate a detection signal reflecting a contact impedance between each of the electrodes and the human body; and
a processing circuit, configured to determine a difference between detection signals corresponding to the two electrodes for evaluating the physiological signal.

17. The circuit of claim 16, wherein the processing circuit includes a gain circuit, and the detection signals corresponding to the two electrodes are designated as an input of the gain circuit for differential amplification.

18. The circuit of claim 17, wherein the AC excitation source is coupled to a corresponding electrode through a resistance voltage divider, and the resistance voltage divider divides a voltage of the AC excitation source to generate a corresponding detection signal.

19. The circuit of claim 18, wherein a frequency of the AC excitation source is greater than 250 Hz.

20. The circuit of claim 19, wherein a ratio of an impedance of the resistance voltage divider at the frequency of the AC excitation source to an impedance of the resistance voltage divider at a frequency less than 400 Hz is not greater than 1.6.

21. The circuit of claim 19, wherein impedances of two resistance voltage dividers at the frequency of the AC excitation source are equal.

22. The circuit of claim 17, wherein the processing circuit further includes an analog-to-digital converter disposed at an output end of the gain circuit, and the analog-to-digital converter is configured to perform an analog-to-digital conversion on the physiological signal and the detection signals.

23. The circuit of claim 22, wherein the processing circuit further includes a low-pass filter, and the low-pass filter is configured to extract the physiological signal that is analog-to-digital converted by the analog-to-digital converter; and
the processing circuit further includes a band-pass filter or a high-pass filter, and the band-pass filter or the high-pass filter is configured to extract a difference between the detection signals that are analog-to-digital converted by the analog-to-digital converter.

24. The circuit of claim 16, wherein the AC excitation source is further configured to provide a second excitation signal at a different frequency from that of the excitation signal to generate a second detection signal, and the second detection signal is configured to reflect a contact impedance between each of the two electrodes and the human body at another frequency.

25. The circuit of claim 24, wherein the processing circuit evaluates body composition information of the human body based on the detection signals and the second detection signal.
